# EUROPEAN PATENT SPECIFICATION

(11) **EP 2 388 018 B1**
(45) Date of publication and mention of the grant of the patent: **13.04.2016**
(21) Application number: 11380004.9
(22) Date of filing: 25.01.2011
(51) Int. Cl.: A61L 2/18

(54) **Portable ozonized liquid generator**
Tragbarer Generator für Ozonisierte Flüssigkeit
Générateur portable de liquide ozonisé

(30) Priority: 27.01.2010 ES 201000091
(43) Date of publication of application: 23.11.2011
(73) Proprietor: Sánchez Álvarez, RIcardo, 49032 Zamora (ES)
(72) Inventor: Sánchez Álvarez, RIcardo, 49032 Zamora (ES)

(56) References cited:
- WO-A1-01/58500
- WO-A2-02/054971
- ES-U- 1 065 660
- US-A- 5 625 915
- US-A1- 2002 127 158

## Description

### OBJECT OF THE INVENTION

The present invention relates to a portable ozonized liquid generator device designed for its use either in an autonomous way or connected to other devices, in order to carry out medical and similar treatments. A method of producing ozonized liquid is also disclosed.

The present invention also provides a control of the quantity of the dissolved ozone, continuously checking the dilution, in order to guarantee the stability of the level of ozonized liquid.

The object of the invention is, thus, to provide a new, compact, portable and completely automatic device, suitable for ozonizing various liquids. A method of production of the ozonized liquid using said device is also disclosed. The method allows for the use of the device with minimal user attention. The plurality of connectors in the input and in the outputs facilitate the connection of the device with a handpiece or others devices.

### BACKGROUND OF THE INVENTION

There are several known methods employed in the field of medicine in order to carry out disinfection, hygiene or cleaning such as antiseptics in order to treat injuries. These treatments are based on applying different, more or less effective products, which may have contraindications or may leave various residues.

Alcohols, hypochlorite, chlorhexidine and the most commonly used povidone iodine are found among those products; the latter providing the drawback that, by its features, it can have interactions with other substances or present allergic reactions, such as intolerance to iodine or iodine containing medicaments.

As an oral solution, it is contraindicated for infants up to 30 months and for people suffering thyroidal disorders. The substance is used manually, applying a few drops directly to the affected area, both prior to and after the treatment, but not during the treatment itself. This can favour pathogen proliferation during an intervention process. The method developed by using the device of the present invention, the application during the whole process of the intervention can be realised.

Furthermore, the effects of the ozone, in gas or diluted liquid form, in medical treatments as a virucide, bactericide and fungicide are known. The object has been to develop a new portable, compact and autonomous easy to use device to cover the needs for applying ozonized liquid in diverse possible treatments.

U.S. patent 6153151 describes a system and a method for generating ozone for use in open or closed loop process applications using a fluid, including a water storage tank supplied by an electromagnetic flux unit connected to the water supply line; for exposing water supplied to the tank to an electromagnetic field thereby magnetically polarizing contaminants and dissolved solids present in the water; it is also included an apparatus for producing highly pure oxygen from ambient air for use as a feed gas in generating ozone; a corona discharge ozone generator for producing high purity ozone from a highly pure oxygen feed gas; including also a rapidly rotating shear impeller, for injecting ozone in the water. It also includes an apparatus for measuring the concentration of dissolved ozone present in the water; and a microprocessor based controlled for controlling the system to reach and maintain the suitable ozone concentration levels, depending on the application.

The microprocessor according to US6153151 controls the ozone concentration sensor only, but not the rest of the system. ;

Also, in the water supply line an electromagnetic field is inserted in order to make a magnetic polarization of the contaminants, whereas in the device of this invention the liquids do not present impurities, but it does have a filter (9) in the liquid outlet from the blending tank (6).

The water supply line also requires its connection to a suitable water source, whereas the device of the present invention works with different liquids and it does not need a water source for functioning

The ozone concentration sensor of the tank is connected to its walls, to the outlet of the ozonized water and to the ozone injector, which is inconvenient when emptying the tank. In the device of the present invention, all the tank connections go through the connector of the cap (5), this allows an easy use for its cleaning and maintenance. It also has an illumination system for the tank (10).

The tank of the aforementioned patent does not present an outlet for gases to the exterior, which leads to pressure excess in the tank which can make the pressure of the tank equal to the pressure of the oxygen concentrator, leading to inaccurate production of the blending. The device of the present invention presents a conduit for the remaining gas outlet to the connector (24), which can be used to connect other devices (30) or an ozone destructor (25).

The ozone concentration sensor in the referred patent is directly connected to the tank. This can generate erroneous measurements if the dissolved gas stratification is produced when there are no movements. In the device of the present invention the ozone sensor (16) is connected to a recirculating pump (17), which generates a constant movement of the liquid and provides higher measurement accuracy.

US4282172, ES1056660, WO02054971 further disclose various ozonising systems.

This design does not present an electronic control of the process, it thus needs to be connected to a suitable water source without being able to work in an autonomous or independent way. The device of the present invention works with different liquids.

Moreover, the tank of the U.S. patent 4282172 does not present level sensors, the conduits do not access through the cap to the tank to allow a better manipulation.

It does not present illumination either.

The controller for the ozone quantity in the ozonized water outlet cannot guarantee the levels of dissolved ozone.

It does not allow an easy connection or an easy mobility of the device, either.

WO 00/27760 describes a method for treating water with an ozonizing gas operating under elevated pressure. The elevated pressure is obtained by means of a gas source or a turbine. The elevated pressure of the treated water is optionally used to dispense the treated water. The turbine may be used to obtain particularly fine bubbles of a gas in a liquid in a tight container.

The apparatus of said patent WO 00/27760 does not include a microprocessor that governs and controls all the process.

The device operates under elevated pressure and does not present a humidity drier between the oxygen source and the ozone generator. The device of the present invention comprises a drier (2) between the connector (1) and the compressor (3), the device being operable at low pressures.

The apparatus of said patent comprise conduits and sensors distributed along the tank. In the device of this invention, the conduits and the sensors are in the cap.

The aforementioned patent neither presents illumination of the tank, nor an automatic outlet system for the ozonized liquid. The device of the present invention has an outlet pump (18), an outlet sensor (19) and two connectors (20-21) for the ozonized liquid supply being completely automatic.

All cases present the drawback of lacking a precise electronic control to supervise all the systems for the complete functioning of the device. They lack a drier at the compressor inlet and in the ozone generator. The conduits are connected at any side of the tank; in the device of this invention, the connections of the tank (6) are made by means of a connector (5) situated in the cap to facilitate the extraction and be able to carry out a more adequate maintenance The devices of the aforementioned patents do not present an illumination system (10) of the tank (6); they do not present a liquid inlet selection (15) with a double filling system of the liquid to be ozonized; they need to be connected to a water source supply, forced to be in a concrete place and preventing the fast mobility of the device. The ozone sensors are ORP (Redox Potential) type sensors and they are in contact with the liquid in order to measure it. In the device of this invention, the sensor is photometric not being in contact with the liquid, which leads to a greater hygiene in the process. These ORP (Redox Potential) sensors measure in an indirect way leading to a lack of precision, which does not happen with the photometric method. The ozonisation control system of the present invention is connected to the tank, and the liquid circulation is forced in order to achieve that there are not different diluted ozone levels when the liquid of the tank does not move. In the tank filling system of the cited patents the sensors are level sensors and they are in contact with the liquid; in the device of this invention the level sensor (7) is a proximity sensor to achieve a greater hygiene, because it is not in contact with the liquid. The device of the present invention is designed to carry out medical treatments, which require process control and a maximum hygiene.

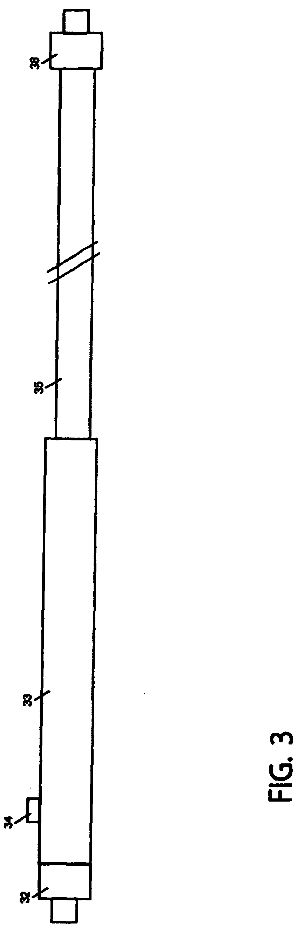

The device of this invention described in Figure 3 comprises a handpiece (31) allowing for its independent functioning.

### DESCRIPTION OF THE INVENTION

The present invention relates to a portable and compact apparatus. A method of producing ozonized liquid, which can be distilled water, serum, oils, gels, alcohols or any other substance that allows to be ozonized in a liquid state, is also disclosed. The apparatus is particularly designed for autonomous use or being connected to other devices in order to carry out medical treatments and similar treatments.

Basically, method of production consists of diluting the ozone gas in several liquids so as to be applied in different medical treatments using an apparatus allowing a suitable charge depending on the type of liquid; through the tank cap allowing manual filling and continuous control of the ozonized liquid production; means to effectively control the liquid inlet, the generation and constant maintenance of the ozonized liquid, as well as the automation of the ozonized liquid outlet supply.

The proposed device for producing the ozonized liquid for medical and/or similar treatments includes eight different units such as a power supply system, a liquid supply system, a gas feeding system, a control system of the ozonisation mixing, an electronic control system, an illumination system of the blending tank, a gas outlet system and a ozonized liquid outlet system. Said units are located inside a hollow casing, and allow for the ozonized liquid production, the ozonised liquid being available in the outlet connectors to facilitate its application by means of suitable systems to carry out treatments.

The power supply system (23) provides the necessary energy for the functioning of all the device components, whether connected to the electrical network or to batteries.

The liquid supply system is designed for introducing different liquids in the blending tank (6), whether manually or by selecting between two ways by means of the selector (15).

The gas feeding system supplies oxygen or ambient air needed for its conversion into ozone and then its blending with the liquid. It comprises a drier in the inlet line (2) through which the air or oxygen loses the possibly contained humidity, passing subsequently to a compressor (3) that sends air or oxygen flux to the ozone generator (4) transforming it into ozone gas and sending it through the connector of the tank cap (5) to the diffuser (8) located inside the blending tank (6).

The system that controls the ozonisation blending controls the amount of ozone dissolved in the liquid of the blending tank (6) by carrying out constant measurement with the ozone sensor (16), which sends the corresponding signals to the electronic control circuit (22), on the liquid of the tank recirculated by the recirculating pump (17) after passing the liquid of the tank through the filter (9).

The electronic control system (22) controls the functioning of all the systems, capturing data from the sensors (7-16-19) and sending suitable signals to the corresponding systems to achieve the correct functioning at all times.

The illumination system of the blending tank (10) lights the blending tank (6), obtaining visual, relaxing and disinfectant effects if a suitable light is used.

The gas outlet system extracts the ozone gas that does not remain in the liquid, it gets out due to the inlet ozone gas pressure produced in the ozone generator (3) in the blending tank (6) through the diffuser (8) causing the remaining gas to leave through the cap connector (5) till the connector (24), so that the device can later be connected to an ozone destructor (25) or other devices (30) in order to carry out medical or similar treatments in the form of gas.

The ozonized liquid outlet system sends the ozonized liquid to the ozonized liquid outlet connectors (20-21) by pump (18) from the blending tank (6) through the filter (9) and the cap connector (5), going through the outlet sensor (19) to the double liquid outlet connectors (20-21). A handpiece hose (31) may be connected to the connector (20) and connector (21) could be reserved to connect other devices or vice versa, depending on the user needs.

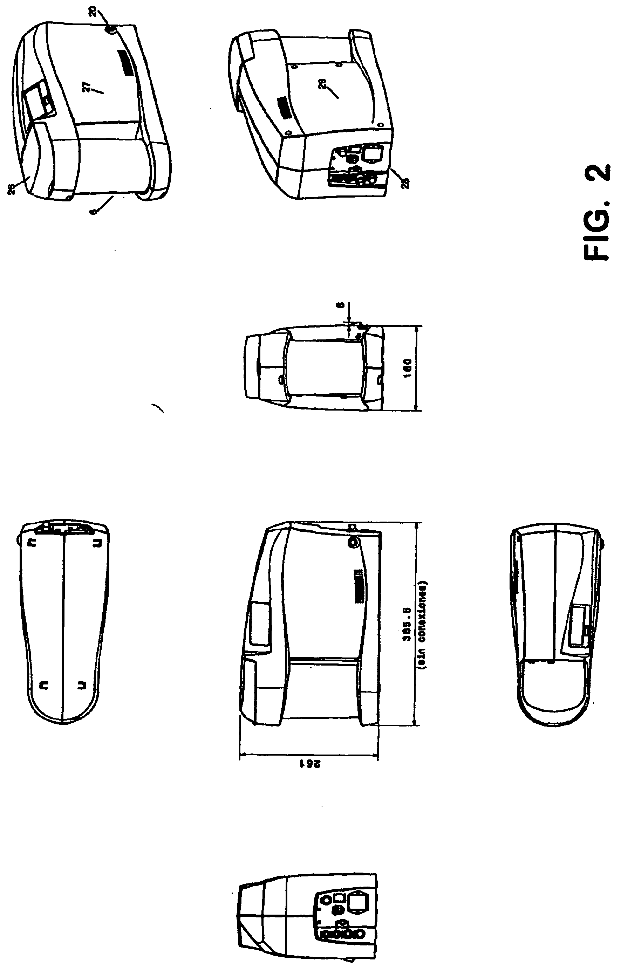

All the systems are mounted inside a casing made of a suitable material (FIG. 2) and being divided into parts to facilitate the assembly of all the components and its maintenance. Said casing comprises a part (26) for the access to the blending tank (6); another part (27) for showing the operating state of system, an on/off button, buttons for the control and a liquid outlet (20); in another part (28) of the casing is located the connecting system with the inlets and outlets of the device ; and a last part (29) is used for closing the assembly. For all this the example of FIG. 2 is presented.

The hose with the handpiece (31) depicted in FIG. 3, allows the direct application of the ozonized liquid, facilitating the work with the device in an autonomous way. It consists of two main parts: the handpiece (33) in a cylindrical shape and made of a suitable material to allow its sterilization, with a button (34) in an end, such that when the button is pushed it allows the flow of liquid to the Luer-look connector (32) to which the appropriate syringe tips may be connected suitable for treatments; and the other is the connection hose (35) made of a flexible material, which allows its sterilization, and a Luerlook connector (36) which may be connected to the outlet connector (20) or (21).

### DESCRIPTION OF THE DRAWINGS

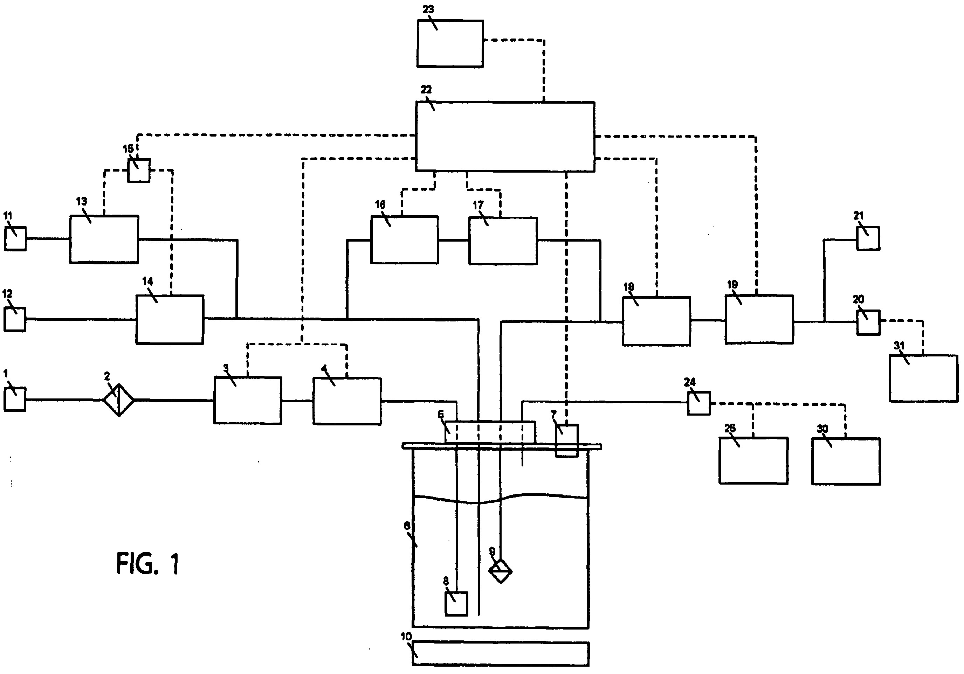

In order to better understand and complete the description of the device of the present invention, three sheets of drawings included as an integrant part of that description, depicting the invention for purposes of illustration and in a non-limiting sense:
- In the first sheet, FIG 1 is a schematic illustration, as a block diagram, of the systems and the necessary components in order to make the device of this invention.

In the second sheet, FIG 2 is a depiction with several perspective views of the casing where the systems that make up the device of the present invention are placed.

In the third sheet, FIG 3 is an illustration of the handpiece hose, with the several components which is made of.

### PREFERRED EMBODIMENT OF THE INVENTION

In view of the above figures, the device of this invention is formed of a casing, which although may have any geometric configuration, the depicted one provides easy handling due to its size and a general view of the tank, even though their materials, shape, size and disposition may vary. Components constituting the distinct systems, in which the device of the present invention is divided into, are placed in said casing.

The proposed method provides, in a completely autonomous way, both the production of the ozonised liquid and the technical system needed for applying the ozonized liquid, requiring minimal attention from the user for its utilization.

The loading process allows to use several liquids and depending on the type of liquid, the inlet may be selected if the liquid is not under pressure. Once the corresponding inlet selector (15) has been activated an inlet pump (13) takes up the liquid and fills the blending tank (6) up to its maximum level, controlled by the level sensor (7), which is a proximity sensor so as not to be in contact with the liquid. Said sensor sends a signal to the electronic control circuit (22) that istransmited through the inlet selector (15) to the inlet pump (13). If a liquid source under pressure is provided, it is selected in the inlet selector (15) and an electro-valve (14) will control the filling of the blending tank (6) up to its maximum level, such that the level sensor (7) sends a signal to the electronic control circuit (22) that will transmit it through the inlet selector (15) to the electro-valve (14), which will allow or cut the flow of the liquid thereby controlling the filling of the blending tank (6). The system also allows the manual filling due to the configuration of the conduit inlets and sensors through the cap connector (5) in the blending tank (6); as well as the easy removal of the cap.

The ozone generator is provided with a connector (1) that allows its external connection to an oxygen concentrator or to a medical oxygen source, said gas lead through a drier (2) to a compressor (3) that generates a constant flux that arrives to the ozone generator (4). This corona discharge type generator has ceramic and titanium electrodes providing high efficiency production of ozone and long durability. The ozone produced should have a suitable amount, so that, when diluted, there will be a sufficient amount in the liquid to achieve medical properties; the produced ozone is introduced in the blending tank (6) through the cap connector (5), and the blending of ozone with the liquid to be ozonized is carried out with the diffuser (8). The blending control system sends a signal to the electronic control circuit (22) about the amount of ozone present in the liquid , which then sends the orders to the compressor (3) and to the ozone generator (4) for their functioning.

The group where the blending of the liquid with the gas takes place is composed of a blending tank (6), a connector placed in the cap (5), a diffuser (8), a level sensor (7), an outlet filter (9) and is controlled by the electronic control circuit (22). It is designed to allow an easy manipulation in order to carry out the maintenance and cleaning tasks; the blending tank (6) is filled with the liquid that will be ozonized up to the maximum level that is controlled by the level sensor (7), and the ozone gas gets out in bubble shape through the diffuser (8) producing the blending with the liquid. The ozone sensor (16) sends the corresponding signal to the electronic control circuit (22), which then sends the necessary signals to control the filling of the tank. As a consequence, liquid ozonisation will always be available for the user. The cap connector (5) allows the way of the different conduits to the blending tank interior (6), making possible an easy manipulation of the tank for its extraction in case of a breakdown or if maintenance tasks are needed.

With the blending tank illumination (10), an aesthetic effect in the blending tank (6) is aimed to achieve, as well as a relaxing visual effect, and being also useful as a disinfectant if suitable light is used.

By means of the pressure escape or the escape of the remaining ozone gas after blending, the ozone gas that has not been blended is led through the cap connector (5) to the outlet connector (24) where a device (30) may be connected in order to carry out treatments with ozone gas, having an ozone gas inlet and a gas outlet to be connected to an ozone destructor, or to be connected directly to an ozone destructor (25) to transform the ozone into oxygen, avoiding the ozone gas emission to the exterior and its possible adverse consequences.

In the control process of the ozonisation blending the level of ozone dissolved in the liquid of the blending tank (6) is controlled through the ozone sensor (16) that receives the ozonized liquid from the recirculating pump (17). With this system, the liquid is continuously circulated, in this way the level of dissolved ozone is controlled in a more effective way. The ozone sensor (16) sends the control signal to the electronic control circuit (22) which sends the relevant control signals to the compressor (3) and to the ozone generator (4). The measurement method is a photometric method, such that the sensor is not directly in contact with the ozonized liquid, which leads to a greater hygiene in the process and a greater accuracy in the measurement.

The ozonized liquid outlet process starts with the blending tank (6) filter (9) through which the ozonized liquid gets out via the cap connector (5) to the outlet pump (18) and driven to the outlet connectors (20-21). The outlet probe (19) sends the corresponding signal to the electronic control circuit (22) which sends the control signal to the outlet pump (18); the electronic control system (22) works in an automatic way and controls the liquid outlet to the connectors (20-21), depending on the ozone level in the liquid of the blending tank (6). The handpiece hose (31) may be connected to the outlet connector (20) and the connector (21) may serve as a spare connector to connect to other devices or vice versa, depending on the user's needs.

By means of a microprocessor in the electronic control circuit (22), the electronic control process is in charge of controlling all the processes that contributes to the good working of the device, receiving the signals of the sensors (7-16-19) and sending the control signals to the rest of the components, so that they appropriately carry out their functioning. The electronic control circuit (22) is also connected to the power supply system (23), which provides the necessary energy for the general working of all the device components, whether connected to the electrical network or to batteries, in order to facilitate the autonomous working and the portability of the device to be able to transport it and install it at any place to carry out the treatments.

## Claims

1. Portable generator device of ozonized liquid for its independent use or connected to other apparatus to carry out medical treatments, comprising:
- liquid inlets (11, 12),
- a selector (15) for selecting a liquid inlet for ozonisation of a corresponding liquid,
- a blending tank (6) for ozonisation deliverable via said selector (15),
- an ozone generator (4) for generating ozone to be introduced into said blending tank (6) for liquid ozonisation,
- an air or oxygen inlet (1) for introducing air or oxygen into the device to be transformed into ozone,
- said inlet (1) being connected to a compressor (3) through a drier (2) upstream of said ozone generator (4),
- outlets for the ozonized liquid (20, 21),
- an outlet pump (18) for pumping the ozonised liquid out of said blending tank (6) to said outlets (20, 21),
- an outlet sensor (19) for controlling the outflow of the ozonised liquid,
- a connector (20) for attaching a handpiece hose (31) to an outlet for the ozonised liquid of said device,
- an ozone gas outlet connector (24) attached to a cap connector (5) on a cap of said blending tank (6) for connection to an ozone gas destructor (25) or for external devices,
- an illumination system (10) of the blending tank (6),
- a power supply system (23) that allows autonomous utilization of said device,
- an electronic control circuit (22) of said device,
- a system for permanently controlling the ozonisation level of the blending tank (6).

2. Portable device according to claim 1, **characterized in that** it is provided with a casing to contain all the components of said device.

3. Portable device according to claim 1, **characterized in that** the ozonisation level of the tank is controlled by means of an ozone sensor (16) and a recirculating pump of the ozonized liquid (17), to enable proper and continuous measurement of the amount of ozone diluted in the liquid of the blending tank (6).

4. Portable device according to claim 1, **characterized in that** said illumination system (10) of the blending tank (6) provides an aesthetic effect in the tank liquid.

5. Portable device according to claim 1, **characterized in that** said power supply system (23) allows for the device to be connected to an electrical circuit or to batteries, being possible the autonomous use of said device.

6. Portable device according to claim 1, **characterized in that** said humidity drier (2) placed in the inlet line between the connector (1) and the compressor (3) preventing the entry of humidity in the ozone generator (4) as well as the formation of unwanted substances.

7. Portable device according to claim 1, **characterized in that** the device is provided with two liquid inlet connectors (11,12), one of said connectors (11, 12) is connected to an inlet pump (13) in order to take up the liquid to be ozonized and the second connector (12, 11) is connected to an electro-valve (14) for the inlet of liquid under pressure, both of them selectable by the inlet selection device (15), said connectors together with the control circuit and the level sensor (7) control the liquid level in the blending tank (6).

8. Portable device according to claim 1, **characterized in that** said outlet sensor (19) detects the need for supplying liquid to the outlets (20-21).

9. Portable device according to claim 1, **characterized in that** said cap facilitates the access to the interior of said blending tank (6), allowing the manual filling and interior cleaning thereof..

## Patentansprüche

1. Tragbare Generatorvorrichtung für ozonisierte Flüssigkeit zur unabhängigen Nutzung oder mit anderen Geräten, um medizinische Behandlungen durchzuführen, bestehend aus:
- Flüssigkeitseinlässe (11,12),
- einem I (15) zur Auswahl eines Flüssigkeitseinlass für die Ozonisierung einer entsprechenden Flüssigkeit,
- einem Mischtank (6) für die Ozonisierung über den besagten Selektor (15),
- einem Ozongenerator (4) zur Erzeugung von Ozon, um in den besagten Mischtank (6) eingeführt zu werden, um die Flüssigkeit zu ozonisieren,
- einem Luft- oder Sauerstoffeinlass (1) zum Einführen von Luft oder Sauerstoff in die Vorrichtung, um in Ozon verwandelt zu werden,
- besagte Einlass (1) über einen Trockner (2) gegen den Strom des besagten Ozongenerators (4) an einen Kompressor (3) angeschlossen ist,
- Auslässe für ozonisierte Flüssigkeit (20,21),
- einer Auslass pumpe (18), zum Pumpen der ozonisierte Flüssigkeit aus dem besagten Mischtank (6) zu den besagten Ausgängen (20,21),
- einem Auslasssensor (19) zum Kontrollieren des Ausgangsstroms der ozonisierten Flüssigkeit,
- einem Stecker (20) zum Befestigen eines Schlauchs des Handstücks (31) an einem Auslass für die ozonisierte Flüssigkeit der besagten Vorrichtung,
- einem Stecker für den Auslass von Ozongas (24), der mit dem Stecker (5) der Abdeckung an einer Abdeckung des besagten Mischtanks (6) verbunden ist, für den Anschluss an einen Ozongaszerstörer (25) oder externe Vorrichtungen,
- einem System zur Beleuchtung (10) des Mischtanks (6),
- einem Netzteilsystem (23), mit dem sich die besagte Vorrichtung alleinstehend nutzen lässt,
- einem elektronischen regelkreis (22) der besagten Vorrichtung,
- einem System zur dauerhaft Controlelling des Ozonisierungsniveaus des Mischtanks (6).

2. Tragbare Vorrichtung gemäß Anspruch 1, die sich **dadurch kennzeichnet, dass** sie mit einem Gehäuse versehen ist, um alle Komponenten der Vorrichtung besagten zu enthalten.

3. Tragbare Vorrichtung gemäß Anspruch 1, die sich **dadurch kennzeichnet, dass** das Ozonisierungsniveau des Behälters mithilfe eines Ozonsensors (16) und einer Pumpe zum Umwälzen der ozonisierten Flüssigkeit (17) kontrolliert wird, um die richtige und ständige Messung der Menge an Ozon, das in der Flüssigkeit im Mischtank(6) gelöst ist, zu ermöglichen.

4. Tragbare Vorrichtung gemäß Anspruch 1, die sich **dadurch kennzeichnet, dass** das besagte System zur Beleuchtung (10) des Mischtanks (6) liefern ästhetisch Wirkung auf die Flüssigkeit im Tank.

5. Tragbare Vorrichtung gemäß Anspruch 1, die sich **dadurch kennzeichnet, dass** das besagte Netzteilsystem (23) es ermöglicht, dass die Vorrichtung an einen elektrischen Schaltkreis oder an Batterien angeschlossen wird, so dass die selbstständige Verwendung der besagten Vorrichtung möglich ist.

6. Tragbare Vorrichtung gemäß Anspruch 1, die sich **dadurch kennzeichnet, dass** besagten Luftfeuchtigkeit Trockner (2) Einlassleitung zwischen dem Stecker (1) und dem Kompressor (3) das Eindringen von Feuchtigkeit in den Ozongenerator (4) sowie die Bildung unerwünschter Stoffe verhindert.

7. Tragbare Vorrichtung gemäß Anspruch 1, die sich **dadurch kennzeichnet, dass** die Vorrichtung mit zwei Steckern (11,12) am Flüssigkeitseinlass versehen ist, von denen einer der besagten Stecker (11,12) an eine Einlassspumpe (13) angeschlossen ist, um die Flüssigkeit auszusaugen, die ionisiert werden soll, und der zweite Stecker (12,11) an ein Elektroventil (14) für den Einlass von Flüssigkeit unter Druck angeschlossen ist und beide Stecker von der Einlasswahlvorrichtung (15) ausgewählt werden und die besagten Stecker zusammen mit dem Steuerkreislauf und dem Niveausensor (7) das Niveau im Mischtank (6) kontrollieren.

8. Tragbare Vorrichtung gemäß Anspruch 1, die sich **dadurch kennzeichnet, dass** der besagte Auslasssensor (19) den Bedarf feststelle, Flüssigkeit an die Ausgänge zu liefern (20,21).

9. Tragbare Vorrichtung gemäß Anspruch 1, die sich **dadurch kennzeichnet, dass** die besagte Abdeckung den Zugang zum Inneren des besagten Mischtanks (6) ermöglicht, so dass er manuell gefüllt und von innen gereinigt werden kann.

## Revendications

1. Dispositif portable générateur de liquide ozonisé à utiliser de manière indépendante, ou bien raccordé à d'autres appareils pour réaliser des traitements médicaux, comprenant :
- des entrées de liquide (11,12),
- un sélecteur (15) pour choisir une entrée de liquide pour l'ozonisation du liquide correspondant,
- un réservoir de mélange (6) pour l'ozonisation par le biais de ce sélecteur (15),
- un générateur d'ozone (4) pour produire de l'ozone à introduire dans ce réservoir de mélange (6) pour ozoniser le liquide,
- une entrée d'air ou d'oxygène (1) pour introduire de l'air ou de l'oxygène dans le dispositif afin de le transformer en ozone,
- cette entrée (1) est branchée à un compresseur (3) à travers un sécheur (2) à contre courant du générateur d'ozone (4),
- des sorties pour le liquide ozonisé (20,21),
- une pompe de sortie (18) pour pomper le liquide ozonisé en dehors du réservoir de mélange (6) vers ces sorties (20,21),
- un détecteur de sortie (19) pour contrôler le flux de sortie du liquide ozonisé,
- un connecteur (20) pour fixer un tuyau de la pièce à main (31) à une sortie pour le liquide ozonisé de ce dispositif,
- un connecteur de sortie de gaz d'ozone (24) relié au connecteur du couvercle (5), sur un couvercle de ce réservoir de mélange (6) pour le branchement à un destructeur de gaz d'ozone (25) ou pour des dispositifs externes,
- un système d'éclairage (10) du réservoir de mélange (6),
- un système de source d'alimentation (23) permettant d'utiliser ce dispositif de manière autonome,
- un circuit de contrôle électronique (22) de ce dispositif,
- un système pour contrôler en permanence le niveau d'ozonisation du réservoir de mélange (6).

2. Dispositif portable selon la revendication 1, **caractérisé en ce qu'**il est muni d'une carcasse pour contenir tous les composants de ce dispositif.

3. Dispositif portable selon la revendication 1, **caractérisé en ce que** le niveau d'ozonisation du réservoir est contrôlé par un détecteur d'ozone (16) et une pompe de recirculation du liquide ozonisé (17), permettant de mesurer en continue et de manière adéquate la quantité d'ozone dilué dans le liquide du réservoir de mélange (6).

4. Dispositif portable selon la revendication 1, **caractérisé en ce que** ce système d'éclairage (10) du réservoir de mélange (6) apporte un effet esthétique au liquide du réservoir.

5. Dispositif portable selon la revendication 1, **caractérisé en ce que** ce système de source d'alimentation (23) permet de brancher le dispositif à un circuit électrique ou aux batteries, ce dispositif pouvant être utilisé de manière autonome.

6. Dispositif portable selon la revendication 1, **caractérisé en ce que** ce séchoir d'humidité (2) placé sur la ligne d'entrée entre le connecteur (1) et le compresseur (3) prévient l'entrée d'humidité dans le générateur d'ozone (4), ainsi que la formation de substances indésirables.

7. Dispositif portable selon la revendication 1, **caractérisé en ce que** le dispositif est muni de deux connecteurs d'entrée de liquide (11,12), un de ces connecteurs (11,12) est branché à une pompe d'entrée (13) afin d'absorber le liquide qui va être ionisé, et le deuxième connecteur (12,11) est branché à une électrovanne (14) pour l'entrée de liquide à pression, ces deux connecteurs étant sélectionnés par le dispositif de sélection d'entrée (15), Ces connecteurs, de même que le circuit de contrôle et le détecteur de niveau (7), contrôlent le niveau dans le réservoir de mélange (6).

8. Dispositif portable selon la revendication 1, **caractérisé en ce que** ce détecteur de sortie (19) détecte le besoin de fournir du liquide aux sorties (20,21).

9. Dispositif portable selon la revendication 1, **caractérisé en ce que** ce couvercle facilite l'accès à l'intérieur de ce réservoir de mélange (6), ce qui permet de le remplir manuellement et d'en nettoyer l'intérieur.
